# EUROPEAN PATENT APPLICATION

(11) **EP 0 700 687 A1**
(43) Date of publication of application: **13.03.1996**
(21) Application number: 95109725.2
(22) Date of filing: 22.06.1995
(51) Int. Cl.: A61M 16/00, A61M 16/08, A61M 39/10

(54) **Dual one-way valved respiratory "T" with threaded locking female luer connector**

(30) Priority: 23.06.1994 US 264374
(71) Applicant: Milliken, Ralph A., New City, NY (US)
(72) Inventor: Milliken, Ralph A., New City, NY (US)
(74) Representative: Müller, Hans-Jürgen, Dipl.-Ing.

(57) **Abstract**

The present invention relates to an innovation in the field of, Maximal Inspiratory Pressure (MIP), and more particularly measurement of MIP and dual pressure. The present invention is an improvisation in the Dual One-Way Valved Respiratory "T" connector (7) that is used in such measurements. The innovative dual one way valved "T" piece (7) of the present invention is fitted above the airway limb with a threaded locking female Luer connection (1), the purpose of which is the use of a measuring device therewith and in particular the hand held portable dual range pressure gage, which combination virtually eliminates the problem in leakage in such measurements and therefore resulting in improved accuracy.

## Description

### FIELD OF THE INVENTION

The present invention relates to the field of Dual and Maximal Inspiratory Pressure measurement and virtual elimination of leakage which is essential to the accuracy of such measurements.

### CROSS-REFERENCES TO RELATED APPLICATIONS

My co-pending United States Patent Application Serial Number 08/089/102, entitled Digital Dual Pressure Gage is a cross-reference.

### BACKGROUND OF THE INVENTION

The following art has been found to be related to the field of the present invention but in no way does any of the of the herein cited references anticipate or even suggest the novel advance in the art that is made by the present invention.

U.S. Patent No. 4,506,665, issued to Andrews, et al., entitled Anesthetic Supply Valve and Mask, which discloses an adapter and valve kit for replacement of a conventional anesthetic mask fitting. There are no threaded locking Luer connections disclosed or used in this patent. The connectors are 15, 22 and 26 mm fitting connections, no Luers are disclosed.

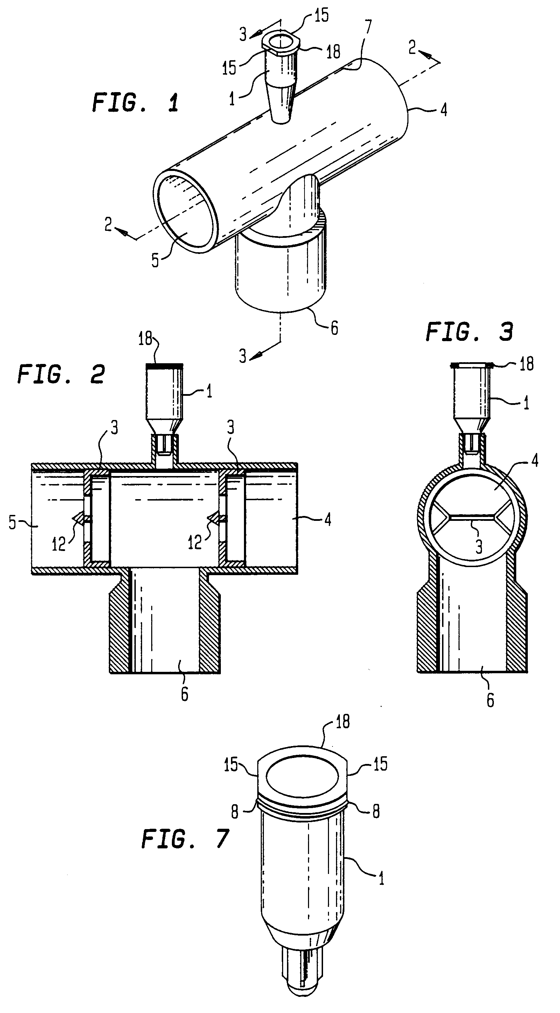

U.S. Patent No. 4,558,708, entitled Device for Sensing Air Flow, which discloses a complicated device for sampling air flow and while it makes a one line general reference to the possible use of Luer connection in only this one instance, it is not mentioned again in the entire patent or claims. However, it has been found to be even further away from the present invention since from the unidentified drawing it is evident that the Luer connector device shown at 32 in Figs. 2,3 and 5 is not a threaded locking female Luer connector as is essential to the present invention and actually teaches away from the disclosure herein. It must further be realized that the air flow of this reference in no way relates to the dual one-way valved respiratory "T" piece, with a threaded locking female Luer connection that is essential to making a leak proof connection to a device such as a dual range pressure gage, either stationary or portable.

U.S. Patent 4,884,460, issued to Norwacki, et al., entitled Device for Sensing Air Flow, which discloses a venturi device for monitoring air flow rate and volume. There is no disclosure of a dual one-way valved respiratory "T" piece with a threaded locking female Luer connection located above the limb of the air flow of the "T" piece..

U.S. Patent No. 5,062,420, issued to Levine, entitled Sealed Swivel for Respiratory Apparatus, which discloses a sealed swivel for a respiratory apparatus which includes an end for engagement with either an endotracheal tube or face mask. Another end, with a telescoping formation is for insertion into a tubular respiratory connector, and has a central portion with a sealing formation defining a swivel track and configured for creating a gas tight seal while permitting relatively free swiveling action. There is no disclosure of a threaded locking female Luer connector.

DHD DIE Molding Healthcare Division Product Catalogue, 601, 000-001D, 41392, 1992, Die Molding Corporation, which on page 4 discloses a NIF-TEE, Non-Rebreathing "T:" Piece, DHD33-3500. This device is directly related to the present invention, however, on the top portion of the "T", a proprietary non-standard connector is provided. This connector is exactly about which the present invention, is the improvement.

This kind of propreatary connector is what causes the devastating leakage and causes the need for the myriad of connectors and tubes in a hospital department.

Intersurgical Incorporated, Accessories and Connectors, 22mm. 15mm and 8.5 mm, Information Pack 5-9004, which discloses on pages 9, 10 and 11, "T" piece with various connectors on top and others with no dual one way valved "T" pieces. However, no dual one-way valved "T" pieces with a threaded locking Luer connector is shown. Baxter Respiratory Therapy Systems, 1989 Hospital Supply Guide, which discloses a disposable "T" piece, all such valves are disposable after use, on page 7, see a disposable "T" with 15 mm base, 2 one-way valves and a thermometer connection on top. There is no disclosure of the type of connector that is part of the present invention.

The Isotherm™ Breathing System Catalogue which discloses a device that delivers gas under pressure with the intent of providing artificial ventilation for a baby. There are no threaded locking female Luer connections disclosed, only proprietary tapered connections which are subject to the leakage that the present invention corrects.

### SUMMARY OF THE INVENTION

The present invention is a dual valved one-way tracheal non-rebreathing "T" piece that is fitted, above the airway limb on the top position between the two one-way valves, with a threaded locking female Luer connector. While tLuer connections have been used in the medical art and have 5 been standardized by ANSI No. MD70. 1983, the innovation of the present invention is the use of the female threaded locking connector. It is absolutely necessary that the male connector has threading on the Luer cap ridge and said cap ridge has to have two opposing flat edges on said cap ridge to accomplish the locking and virtually eliminate leakage. It is also important that the Luer connector be located above the airway limb of the "T" piece between the two one-way valves.

The necessity of this type of dual one-way non-rebreathing "T" piece is that it is not to be permitted to have the patient rebreath their exhausted and contaminated breath as his breathing ability is being monitored and MIP is being performed.

All of the presently available "T" pieces that come from the medical supply houses which have take off connectors in that area between the two one-way valves, have proprietary fittingsof an unlimited variety, which require each hospital department to have in stock an unlimited supply of tubing, connectors and adapters unless they purchase all of there supplies from the same supplier, which is highly unlikely. But this is the goal that the suppliers have by using various proprietory connectors instead of the Luer connector of the present invention.

An even more important consideration is, all of these proprietory connections that are located in the area above and between the two one-way valves of the "T" pieces, is that they are all subject to leakage, which prevents accuracy in the measurement of a patient's ability to breath weaned from artificial breathing means, upon which the life of the patient depends.

MIP is commonly used as one of the predictors of a patients readiness to be weaned from ventilatory support. The measurement is made by the patient inspiring against an occluded airway. Factors determining the MIP value at the time of the airway occlusion, duration of occlusion and respiratory muscle strength. A dual one-way valved "T" piece is now used exclusively such dual pressure reading in MIP. This type of valve allows patient to exhale without the danger of reinhaling his contaminated exhausted breath.

As previously stated, a leak proof connection for pressure measurements from a dual one-way valved "T" piece, has always been a problem and has always made such readings suspect. The present invention has essentially eliminated this problem in the measurement of the positive and negative breathing pressure of a patient. The threaded locking Luer connection supplied devices of the present invention offer a positive seal, eliminating accidental disconnections and more important they effectively eliminate the leakage problem in the pressure measurements that are present with the push-on connectors.

The threaded locking connections of the present invention, forming the dual one-way valved "T" piece of the present invention, makes an ideal connection for permanently installed devices as well as, the hand held portable dual range digital pressure gage that is disclosed in my co-pending U.S. PatentApplication, SN 08/089.102 filed 07/12/93. This makes an ideal combination for all respiratory professionals. The portable dual range digital device, as mentioned supra, has a male Luer connection and the only thing that a respiratory professional has to do is to carry the above described gage in his pocket and a Luer fitted tubing. He can immediately connect to the "T" piece at the bed side and obtain a reliable positive and/or negative pressure of the patient ventlatory effects.

### OBJECTS OF THE INVENTION

The object of the present invention is to provide a dual one-way valved respiratory "T" piece that is fitted with a threaded lockable female Luer connection above the airway limb of said "T" between the valves.

Another object of the present invention is to supply a dual one-way valved respiratory "T" piece that is fitted with a threaded lockable female Luer connection above the airway limb of said "T" between the valves that allow a positive connection with all measuring devices, virtually eliminating the danger of leakage.

A further object of the present invention is to provide a dual one-way valved respiratory "T" piece that is fitted with a threaded lockable female Luer connection above the airway limb of said "T" between the valves that will allow the elimination of a myriad of connections, tubing and adapters.

Various other objects, advantages and features of the present invention will become apparent to those skilled in the art from the previous and following discussions, taken in conjunction with the accompanying drawings, which constitute part hereof.

### BRIEF DESCRIPTION OF THE DRAWINGS

**Fig. 1** is a perspective view of the dual one-way valved respiratory "T" piece with the threaded lockable female connector thereon of the present invention.
**Fig. 2** is a sectional view taken on line 2---2 of **Fig. 1**
**Fig. 3** is a sectional view taken on line 3---3 of **Fig. 1**.
**Fig. 4** is a side view of the threaded lockable male Luer connector.
**Fig. 5** is a side view showing only the threaded lockable female Luer connector
**Fig. 6** is a perspective view of the dual one-way valved "T" piece that is connected to the digital dual range pressure gage of my co-pending application to which was previously referred.
**Fig. 7** is a perspective view of the threaded lockable male Luer connector, specifically showing the threaded cap ridge and the opposing flat sides of said cap, both of which are essential to the present invention.

### DESCRIPTION OF THE PREFERRED EMBODIMENT OF THE PRESENT INVENTION

The MIP, or Maximal Inspiratory Pressure is taken utilizing the "T" piece of the present invention and is shown in **Fig. 1** at 7, where the patient's breath enters at the inspiratory end at 4 and travels through two one-way valves at 3 with directions caps 12 as shown in **Fig. 2** and expiration occurs at 5. Any measurement device, such as a manometer, may be connected at 6. The threaded locking female connector 1 of the present invention is shown in delail in **Fig, 7 and** in **Figs**. **1, 2, 3, 4 and 6**.

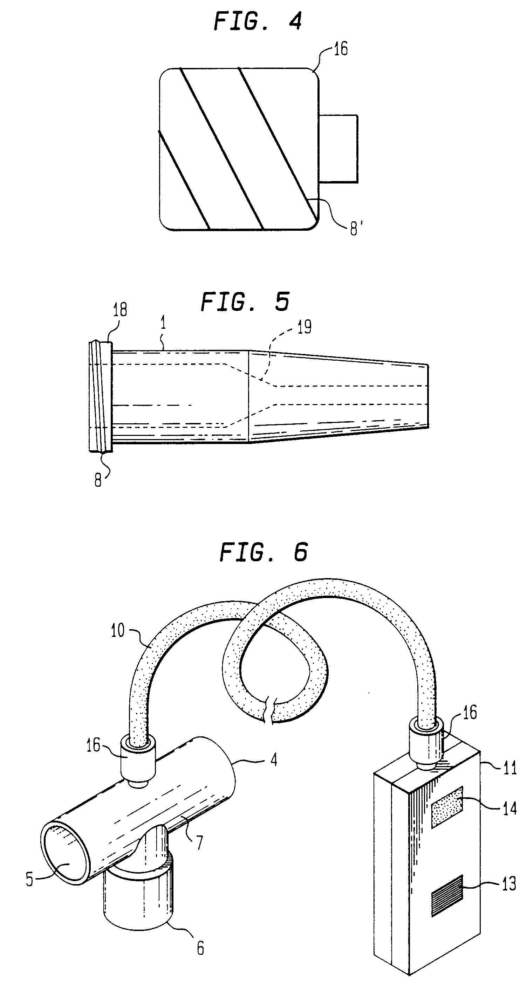

When it is desired to measure both the positive and/or negative pressure of a supported patient, the professional removes a screwed-on protective cap that closes and seals the threaded lockable female connector 1 of the present invention. The professional carries with him a tubing of any predetermined length 10 and the portable hand-held pressure gage of my so-pending U.S. Patent Application SM. 08/089,102, filed 07/12/93 which has a threaded female connector as shown in **Fig. 6** at 11 and is shown connected to the "T" piece 7 of the present invention. The gage 11 has a digital display at 14 and a power on/auto off switch at 13. The threaded lockable female Luer connector 1 is at the top of the "T" piece 7 and the gage 11. The tubing 10 has the male connector 1 of **Fig. 4** at both ends.

The threaded lockable Luer female connection 1 is at the top of the gage 11 and the "T" piece 7 threaded to the male connectors of tubing 10 at both ends.The threads on the threaded female Luer connection are shown at 8 in **Figs. 2, 3, and 5** and the inner tubing is shown at 9 in the same figures.

The female connector of the threaded locking Luer connector that is an essential part of the present invention is shown in **Figs. 5** and **7**, wherein the threaded collar is shown at 15, with the essential threads on said collar shown at 8 and the flat opposing sides of collar 18 are shown at 15. These fiat sides are essential to the locking feature of the threaded Luer connector of the present invention which is responsible for the virtual elimination of leakage in the measurement of respiratory patients.

The over-all purpose of the present invention is to give the professional the ability to take life sustaining measurements with the accuracy needed to properly sustain such life. This is accomplished by the present inventions virtual elimination of measurement leakage that has plagued the respiratory professionals since the inception of such measurements.

While the above description contains many specificities, the reader would not construe these as limitations on the scope of the invention, but merely as exemplification of a preferred embodiment. Those skilled in the art will envision that many other possible variations are within the scoop of the present invention. For example, skilled artisans will readily be able to change the dimensions and the materials of various embodiments. They can make variations on the design of the present invention. Accordingly, the reader is requested to determine the scope of the present invention by the scope of the appended claims and their legal equivalents, and not by the examples that have been given.

## Claims

1. Dual one-way valved respiratory T-piece (7),
**characterized in that**
said T-piece (7) is supplied with a threaded lockable female Luer connector (1).

2. Respiratory T-piece as claimed in claim 1,
**characterized in that**
said female Luer connector (1) is in combination with and adapted to receive a threaded lockable male Luer connection at the end of tubing for connection to measuring devices.

3. Respiratory T-piece as claimed in claim 1 or 2,
**characterized in that**
said threaded lockable female Luer connector (1) is located at the top portion of said T-piece (7), between said dual valves (3) and above the airway limb of said T-piece (7).

4. Connector (1) for a respiratory T-piece as claimed in one of the preceding claims,
**characterized in that**
said female connector (1) is provided with a collar (18) of generally round shape wherein said collar (18) comprises threads (8) and flat sides (15) on opposing sides to afford said combination lockability.
